Europäisches Patentamt

European Patent Office

Office européen des brevets

(11) Veröffentlichungsnummer: **0 190 745**
**A2**

## (12) EUROPÄISCHE PATENTANMELDUNG

(21) Anmeldenummer: 86101502.2

(22) Anmeldetag: 05.02.86

(51) Int. Cl.⁴: **A 61 N 1/16**

(30) Priorität: 08.02.85 DE 3504247

(43) Veröffentlichungstag der Anmeldung:
13.08.86 Patentblatt 86/33

(84) Benannte Vertragsstaaten:
AT BE CH DE FR GB IT LI LU NL SE

(71) Anmelder: Schnaufer, Rolf, Dr.

D-7525 Weil der Stadt(DE)

(72) Erfinder: Schnaufer, Rolf, Dr. Dipl.-Chem.
Hermann-Schnaufer-Strasse
D-72523 Weil der Stadt(DE)

(72) Erfinder: Schweizer, Paul, Dr. Dipl.-Phys.
Hermann-Löns-Strasse 31
D-7032 Sindelfingen(DE)

(74) Vertreter: Patentanwälte Phys. Bartels Dipl.-Ing. Fink
Dr.-Ing. Held
Lange Strasse 51
D-7000 Stuttgart 1(DE)

(54) Flächenhaftes Gebilde zur Abschirmung pathogener radiästhetisch messbarer Strahlung.

(57) Bei einem flächenhaften Gebilde zu einer wenigstens unvollständigen Abschirmung pathogener, radiästhetisch meßbarer Strahlung von Wasseradern, geologischer Verwerfungen, Gitternetzen und dergleichen ist ein Träger vorhanden für ein System, das in der vom Träger definierten Fläche mindestens drei langgestreckte Elemente (1, 2) in regelmäßiger Anordnung mit gleichen Abständen zwischen benachbarten Elementen (1, 2) aufweist. Die einzelnen Elemente (1, 2) haben dabei einen von der Geradlinigkeit nur entsprechend dem Heraustreten des Trägers aus einer ebenen Fläche abweichenden Verlauf. Der Abstand zwischen zwei benachbarten Elementen (1, 2) ist kleiner als 37 cm, wobei die untere Grenze 3 cm beträgt. Je nachdem, wodurch die abzuschirmenden Strahlen hervorgerufen werden, muß der Abstand zwischen benachbarten Elementen ungleich bestimmter Wertbereiche sein.

./...

**Patentanwälte**

European Patent Attorneys

**Phys. H.Bartels
Dipl.-Ing. H.Hink
Dr.-Ing. M.Herr** 745

30.1.1985

Patentanwälte · Lange Straße 51 · D-7000 Stuttgart 1

126 804

3308krw

Zugelassene Vertreter
beim Europäischen Patentamt

Dr. Rolf Schnaufer, 7252 Weil der Stadt

---

Flächenhaftes Gebilde zur Abschirmung pathogener radiästhetisch
meßbarer Strahlung

---

Die Erfindung betrifft ein flächenhaftes Gebilde zu einer wenigstens unvollständigen Abschirmung pathogener radiästhetisch
meßbarer Strahlung von Wasseradern, geologischen Verwerfungen,
Gitternetzen und dergleichen, das die Merkmale des Oberbegriffs
des Anspruchs 1 aufweist.

Die radiästhetisch meßbare Strahlung tritt in der Regel nicht
störend in Erscheinung, weil sie nur in räumlich stark begrenzten Bereichen eine so hohe Intensität hat, daß sie für sensible
Personen eine pathogene Wirkung hervorrufen kann. Sie durchdringt jedoch Mauerwerk und Beton praktisch ohne Intensitätsverlust, weshalb dann, wenn eine Abschirmung erforderlich ist,
diese durch bauliche Maßnahmen, beispielsweise in Form einer
Zwischendecke aus Beton, nicht erreicht werden kann. Es werden
deshalb Matten auf dem Markt angeboten, die als Bettunterlagen
verwendet werden sollen, um den Schlafstellenbereich gegen die
terrestrische Strahlung abzuschirmen, weil die Beeinträchtigung
einer Person durch die terrestrische Strahlung dort am nachhaltigsten ist, wo sich diese Person regelmäßig über einen längeren Zeitraum hinweg aufhält. Diese Matten enthalten in der Regel in einem flexiblen Träger eine metallische Abschirmeinrichtung aus einer Metallfolie oder einem engmaschigen Gewirke
aus Kupferdrähten, das zum Beispiel in eine Wollsteppdecke eingelegt ist.

Viele dieser Matten haben keinerlei abschirmende Wirkung, auch
nicht, wenn sie geerdet sind. Sofern sie eine Metallfolie als
Bestandteil haben, wirkt die Abschirmung nur bis in eine Höhe
von etwa 30 cm. Die Strahlung über Kreuzungsbereichen von Reizz-
zonen wird von allen diesen Matten überhaupt nicht geschwächt.
Es ist aber bekannt, daß gerade die Reizzonenkreuzungen die
pathogene Wirkung verursachen.

Der Erfindung liegt die Aufgabe zu Grunde, ein flächenhaftes
Gebilde zur Abschirmung pathogener, radiästhetisch meßbarer
Strahlung zu schaffen, das diese Nachteile vermeidet und die
Strahlung der Reizzonen und ihrer Kreuzungsbereiche in einem
Maße abschwächt, daß keine geopathischen Belastungen entstehen
oder bestehende geophatische Belastungen verschwinden. Diese
Aufgabe löst ein Gebilde mit den Merkmalen des Anspruchs 1, wobei unter den atmosphärischen Komponenten der terrestrischen
Strahlung diejenigen Strahlen verstanden werden, die auf Grund
von Reflexionen terrestrischer Strahlung zurückzuführen sind
und von oben auf die Erdoberfläche einfallen.

Das erfindungsgemäße Gebilde ist nicht nur als Unterlage für
Betten und sonstige Liegen geeignet, da seine äußere Form und
Abmessungen an die Erfordernisse angepaßt werden können. Es ist
deshalb beispielsweise auch als Sitzunterlage oder Sitzbezug,
und zwar auch für Autositze, als Bodenbelag oder kombiniert mit
einer Antirutschunterlage für Teppiche geeignet. Da das erfindungsgemäße Gebilde auch gegen diejenige radiästhetisch meßbare
Strahlung abschirmt, die von oben her gegen die Erdoberfläche
gerichtet ist und vorzugsweise in der Form von raumgitterartigen Strukturen (Gitternetzen) auftritt.

eignet sich das erfindungsgemäße Gebilde auch für eine Anordnung über der zu schützenden Person, beispielsweise in Form einer Decke, mit der sich diese Person zudeckt.

Für eine Abschirmung terrestrischer Strahlung auf Grund von Wasseradern und der dieser Strahlung zugehörigen atmosphärischen Komponente liegen die Abstände zwischen benachbarten Elementen vorzugsweise in den im Anspruch 2 definierten Bereichen. Für eine Abschirmung gegenüber terrestrischer Strahlung auf Grund von geologischen Verwerfungen und gegenüber der zu dieser Strahlung zugehörigen atmosphärischen Komponente sind Abstände zwischen benachbarten Elementen vorteilhaft, wie sie im Anspruch 3 definiert sind. Es gibt jedoch auch Wertebereiche für die Abstände zwischen benachbarten Elementen, die sowohl eine Abschirmung gegenüber terrestrischer Strahlung auf Grund von Wasseradern und dazugehöriger atmosphärischer Komponente als auch terrestrischer Strahlung auf Grund von geologischen Verwerfungen und der zugehörigen atmosphärischen Komponente besonders vorteilhaft sind. Diese Bereiche sind im Anspruch 4 definiert. Diesen Bereichen kommt insofern einebesondere Bedeutung zu als bei jedem in diesem Bereich liegenden Abstand zwischen benachbarten Elementen die räumliche Form des Gebildes und die geometrische Form seiner Berandung auf die Abschirmungswirkung keinerlei Einfluß haben. So braucht beispielsweise bei einer Verwendung des Gebildes als Bettunterlage nicht darauf geachtet zu werden, daß diese Bettunterlage keine Deformation erfährt. Auch eine örtlich starke Ausbeulung beeinträchtigt die Abschirmungswirkung nicht. Entsprechendes gilt für Knickungen und andere Deformationen.

Vor allem dann, wenn der Träger flexibel ausgebildet ist, sind auch die Elemente vorzugsweise elastisch verformbar. Bei einer vorteilhaften Ausführungsform sind deshalb die Elemente Kupferdrähte, die mit einer Isolation, vorzugsweise einer Lackisolation, versehen sind. Das Material, aus dem die Elemente bestehen, braucht jedoch nicht ein Metall zu sein. Kupferdrähte sind insofern vorteilhaft, als sie sehr biegsam sind und, was wichtig ist, nicht leicht brechen. Unterbrechungen der Elemente sind nämlich zu vermeiden. Die Isolation der Drähte verhindert eine Oxidation und damit die Gefahr des Entstehens von Flecken sowie eines Kurzschlusses durch Nässe. Bei der Verwendung von Lackdrähten kann es sich um handelsübliche Drähte handeln. Der Querschnitt der Drähte kann gering sein, was im Hinblick auf die eventuell gewünschte Flexibilität des Gebildes sowie die Herstellungskosten von Vorteil ist. Vorzugsweise liegt der Drahtquerschnitt zwischen 0,1 mm$^2$ und 0,5 mm$^2$. Gute Ergebnisse wurden mit einem Drahtquerschnitt von 0,15 mm$^2$ erzielt.

Der Träger, der zweckmäßigerweise nicht aus Metall besteht, ist bei einer bevorzugten Ausführungsform ein textiles Gewebe, in das die Elemente, wie die Kettfäden oder Schußfäden, eingebunden sein können.

Besonders vorteilhaft ist es, wenn das Gewebe das Grundgewebe einer Decke ist, da dann der Träger auch die Funktion einer Decke über ehmen kann. Vorzugsweise besteht die Decke wenigtens teilweise, z.B. in der den Flor bilden Polkette, aus naturbelassener Wolle, welche nicht nur die bekannten physiologischen Vorzüge, sondern auch eine gewisse Dämpfungswirkung für Erdstrahlen hat. Bei einer als Decke ausgebildeten Ausführungsform besteht deshalb vorzugsweise das Grundgewebe, in das die Elemente eingebunden sind, aus Baumwolle, während die Polfäden, welche den Flor der Decke bilden, aus reiner Schurwolle bestehen, die vorzugsweise nur gewaschen, nicht aber chemisch ausgerüstet ist. Die eingeschossenen bzw. eingeschärten Elemente sind dabei vorteilhafterweise mit Baumwolle angezwirnt.

Das Gebilde kann auch ein zweites System langgestreckter Elemente aufweisen. Dabei können die Elemente des zweiten Systems parallel zu demjenigen des ersten Systems liegen. Eine solche Anordnung ist beispielsweise dann vorteilhaft, wenn das eine
System die durch Wasseradern hervorgerufene Strahlung, das andere System die durch geologische Verwerfungen hervorgerufenen
Strahlen abschirmt und deshalb die Abstände zwischen den Elementen beider Systeme unterschiedlich gewählt sind. Die Elemente beider Systeme können sich aber auch kreuzen, beispielsweise
im Winkel von 90°. In jedem Fall müssen beide Systeme die Bedingungen des Anspruchs 1 erfüllen.

Ein zweites System ist auch dann von Vorteil, wenn das erste
System bei Deformationen seines Trägers, insbesondere örtlicher
Ausbeulung, wie sie beispielsweise im Gebrauch als Auflage auf
einer Matratze auftreten können, im Deformationsbereich eine
Verminderung der Abschirmwirkung erleidet. Man kann dann mit
Hilfe des zweiten Systems die volle Abschirmwirkung erreichen.
Der Kreuzungswinkel soll hierfür im Bereich von 52°   3% liegen. Die beiden Systeme können je einen eigenen Träger haben,
wobei zweckmäßigerweise die Träger beider Systeme, beispielsweise längs ihres Randes, miteinander verbunden, z.B. zusammengenäht, sind.

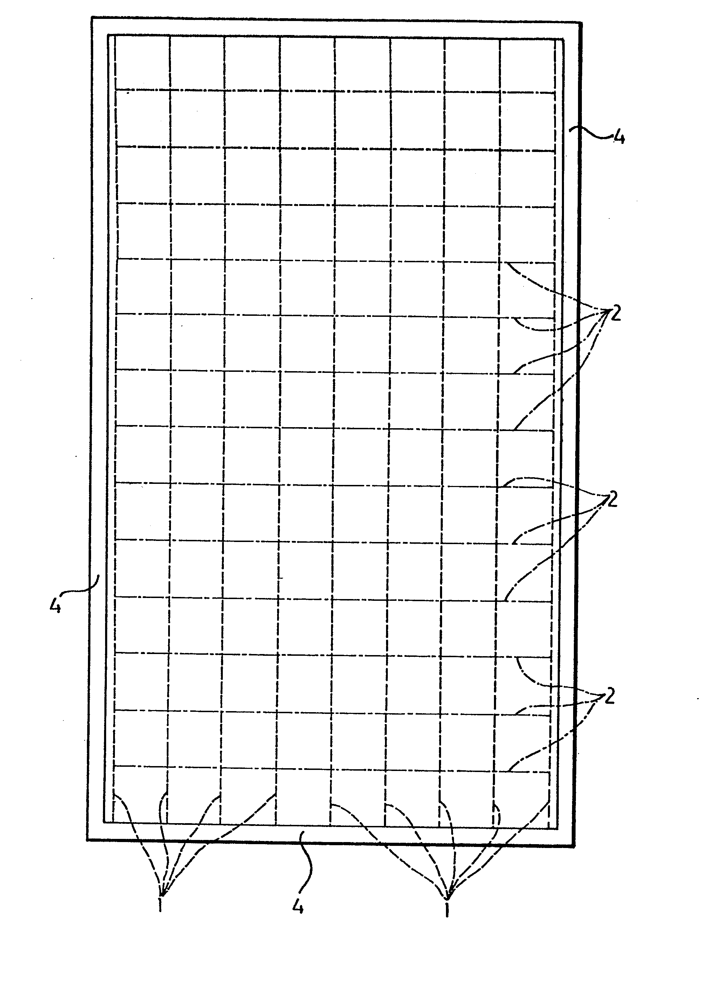

Im folgenden ist die Erfindung an Hand eines in der Zeichnung
dargestellten Ausführungsbeispiels im einzelnen erläutert. Die
einzige Figur zeigt eine nicht maßstabsgerechte dargestellte
Draufsicht auf das Ausführungsbeispiel.

Eine beispielsweise als Bettunterlage verwendbare Decke weist
aus Baumwolle bestehende Kettfäden auf, zwischen die mit Baumwolle angezwirnte Kupferdrähte 1 geschärt sind, und zwar derart, daß der Abstand zwischen zwei benachbarten Kupferdrähten 1, also die lichte Weite zwischen ihnen, 9,2 cm beträgt. Die
Kettfäden bilden zusammen mit ebenfalls aus Baumwolle bestehenden Schußfäden ein Grundgewebe. Statt der zwischen die Kettfäden geschärten Kupferdrähte 1 könnten auch in Schußrichtung
eingeschossene Kupferdrähte 2 vorgesehen sein, wie dies durch strichpunktierte Linien angedeutet ist, wobei die lichte Weite zwischen zwei benachbarten Kupferdrähten ebenfalls 9,2 cm betragen würde. In jedem Falle verlaufen die Kupferdrähte alle parallel zueinander vom Bereich des einen seitlichen Randes der Decke zum Bereich des dazu parallelen anderen seitlichen Randes, haben also alle die gleiche Länge und liegen, wenn das den Träger bildende Grundgewebe in einer Ebene liegt, ebenfalls in einer Ebene.

In das Grundgewebe sind ausschließlich aus gewaschener Schurwolle bestehende Polkettfäden in bekannter Weise eingebunden, wobei das durch die Schlingen der Polkettfäden gebildete Material zur Bildung des Flors der Decke durch Rauhen in Einzelfasern aufgelöst ist.

Die Decke ist längs ihres Randes mit einem textilen Band 4 eingefaßt. Die Kupferdrähte 1 oder 2 haben eine Lackisolation und einen Querschnitt von 0,15 mm$^2$. Sie könnten aber auch blank oder verzinnt sein.

Alle in der vorstehenden Beschreibung erwähnten sowie auch die nur allein aus der Zeichnung entnehmbaren Merkmale sind als weitere Ausgestaltungen Bestandteile der Erfindung, auch wenn sie nicht besonders hervorgehoben und insbesondere nicht in den Ansprüchen erwähnt sind.

Patentansprüche

1. Flächenhaftes Gebilde zu einer wenigstens unvollständigen Abschirmung pathogener, radiästhetisch meßbarer Strahlung von Wasseradern, geologischen Verwerfungen, Gitternetzen und dergleichen mit einem Träger für ein System, das in der vom Träger definierten Fläche mindestens drei langgestreckte Elemente in regelmäßiger Anordnung mit gleichen Abständen zwischen benachbarten Elementen aufweist von denen jedes einen von der Geradlinigkeit nur entsprechend dem Heraustreten des Trägers aus einer ebenen Fläche abweichenden Verlauf hat, wobei der Abstand zwischen zwei benachbarten Elementen kleiner als 37 cm ist, dadurch gekennzeichnet, daß

a) die untere Grenze des Abstandes zwischen zwei benachbarten Elementen (1, 2) 3 cm beträgt und

b) der Abstand zwischen benachbarten Elementen (1, 2)ungleich ist einem Wert im Bereich 18,5 cm $^+$ 3% oder einem ganzzahligen Vielfachen dieses Wertes sowie ungleich einem Wert in den Bereichen 3,8 cm $^+$ 3 %, 8,2 cm $^+$ 3%, 10,75 cm $^+$3%,12,2 cm $^+$ 3% und 13,9 cm $^+$ 3% sowie einem ganzzahligen Vielfachen dieser Werte für eine Abschirmung terrestrischer Strahlung auf Grund geologischer Verwerfungen und der zugehörigen atmosphärischen Komponente dieser Strahlung und ungleich einem Wert im Bereich 7,7 cm $^+$ 3% sowie einem ganzzahligen Vielfachen dieses Wertes für eine Abschirmung terrestrischer Strahlung auf Grund von Wasseradern und der zugehörigen atmosphärischen Komponente dieser Strahlung.

2.  Gebilde nach Anspruch 1, dadurch gekennzeichnet, daß die
    Abstände zwischen benachbarten Elementen in den Bereichen
    von 3,9 cm $\pm$ 3% oder 11,6 cm $\pm$ 3% für eine Abschirmung
    terrestrischer Strahlung auf Grund von Wasseradern und der
    zugehörigen atmosphärischen Komponente liegen.

3.  Gebilde nach Anspruch 1, dadurch gekennzeichnet, daß die
    Abstände zwischen benachbarten Elementen in den Bereichen
    von 5,4 cm $\pm$ 3% oder 16,1 cm $\pm$3% für eine Abschirmung
    terrestrischer Strahlung auf Grund von geologischen Verwerfungen und der zugehörigen atmosphärischen Komponente liegen.

4.  Gebilde nach Anspruch 1, dadurch gekennzeichnet, daß der
    Abstände zwischen benachbarten Elementen in den Bereichen
    von 5,8 cm $\pm$ 3%, 8,0 cm $\pm$ 3%, 9,2 cm $\pm$ 3%, 10,0 cm
    $\pm$ 3%, 10,8 cm $\pm$ 3%, 17,3 cm $\pm$ 3%, 23,9 cm $\pm$ 3%,
    27,7 cm $\pm$ 3%, 29,9 cm $\pm$ 3% oder 32,5 cm $\pm$ 3% liegen.

5.  Gebilde nach einem der Ansprüche 1 bis 4, dadurch gekennzeichnet, daß die Elemente (1,2) elastisch verformbar sind
    und der Träger flexibel ausgebildet ist.

6.  Gebilde nach Anspruch 5, dadurch gekennzeichnet, daß die
    Elemente Kupferdrähte (1,2) sind und mit einer Isolation,
    vorzugsweise einer Lackisolation, versehen sind.

7.  Gebilde nach Anspruch 6, dadurch gekennzeichnet, daß die
    Kupferdrähte (1,2) einen Querschnitt zwischen 0,1 mm$^2$ und
    0,5 mm$^2$, vorzugsweise einen Querschnitt von etwa
    0,15 mm$^2$, haben.

8.  Gebilde nach einem der Ansprüche 1 bis 7, dadurch gekennzeichnet, daß der Träger ein textiles Gewebe ist und die
    Elemente (1,2) in Kettrichtung oder in Schußrichtung verlaufen.

9. Gebilde nach Anspruch 8, dadurch gekennzeichnet, daß die Elemente (1,2) in das Gewebe wie dessen Kettfäden oder Schußfäden eingebunden sind.

10. Gebilde nach Anspruch 9, dadurch gekennzeichnet, daß die im Gewebe angeordneten Elemente (1,2) mit einem Garn, vorzugsweise aus Baumwolle, angezwirnt sind.

11. Gebilde nach einem der Ansprüche 8 bis 10, dadurch gekennzeichnet, daß das Gewebe das Grundgewebe einer Decke ist.

12. Gebilde nach Anspruch 11, dadurch gekennzeichnet, daß das Grundgewebe der Decke aus Baumwolle und die den Flor der Decke bildenden Polkettfäden aus reiner Schurwolle bestehen.

13. Gebilde nach einem der Ansprüche 1 bis 12, gekennzeichnet durch ein zweites System mit langgestreckten Elementen.

14. Gebilde nach Anspruch 13, dadurch gekennzeichnet, daß die Elemente des zweiten Systems eine das erste System kreuzende Anordnung haben.

15. Gebilde nach Anspruch 13 oder 14, dadurch gekennzeichnet, daß der Abstand der Elemente des zweiten Systems die Bedingungen gemäß Anspruch 1 erfüllt und vorzugsweise einen der Werte gemäß den Ansprüchen 2 bis 4 hat.

16. Gebilde nach einem der Ansprüche 13 bis 15, gekennzeichnet durch einen separaten Träger für das zweite System, der mit dem Träger des ersten Systems verbunden ist.

17. Gebilde nach Anspruch 16, dadurch gekennzeichnet, daß der Träger für das zweite System ein Gewebe ist.

18. Gebilde nach Anspruch 14, dadurch gekennzeichnet, daß der Kreuzungswinkel im Bereich von 52° $\pm$ 3% liegt.

- . -

0190745

Dr. ROLF SCHNAUFER ........

Reg.-Nr: 126 804